# EUROPÄISCHE PATENTANMELDUNG

(11) **EP 1 905 440 A1**
(43) Veröffentlichungstag der Anmeldung: **02.04.2008**
(21) Anmeldenummer: 06120196.8
(22) Anmeldetag: 06.09.2006
(51) Int. Cl.: A61K 31/437, A61P 11/00

(54) **Verwendung einer pharmazeutischen Zusammensetzung enthaltend ein Anticholinergikum zur Abtötung von Mikroorganismen und zur Behandlung von Atemwegsinfektionen**

(71) Anmelder: Boehringer Ingelheim Pharma GmbH & Co. KG, 55216 Ingelheim am Rhein (DE)
(72) Erfinder: Die Erfindernennung liegt noch nicht vor
(74) Vertreter: Hammann, Heinz

(57) **Zusammenfassung**

Die vorliegende Erfindung betrifft die Verwendung einer Verbindung der Formel **1** 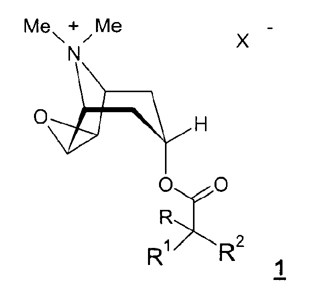 worin
X ⁻
ein einfach negativ geladenes Anion,
R
H, Methyl oder OH
R¹ und R²
gleich oder verschieden, Phenyl oder Thienyl bedeuten,
zur Herstellung eines Arzneimittels zur Abtötung von Mikroorganismen und/oder zur Herstellung eines Arzneimittels zur Behandlung von durch Mikroorganismen verursachten Atemwegsinfektionen.

## Beschreibung

Die vorliegende Erfindung betrifft die Verwendung einer Verbindung der Formel **1** 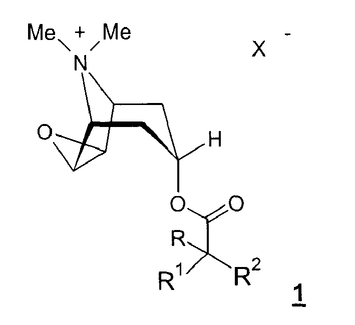 worin
- X ⁻: ein einfach negativ geladenes Anion,
- R: H, Methyl oder OH
- R¹ und R²: gleich oder verschieden, Phenyl oder Thienyl bedeuten,
zur Herstellung eines Arzneimittels zur Abtötung von Mikroorganismen und/oder zur Herstellung eines Arzneimittels zur Behandlung von durch Mikroorganismen verursachten Atemwegsinfektionen.

Weiterhin betrifft die vorliegende Erfindung eine pharmazeutische Zusammensetzung einer Verbindung der obigen Formel **1**, gegebenenfalls in Form der einzelnen optischen Isomeren, Mischungen der einzelnen Enantiomeren oder Racemate, wobei die pharmazeutische Zusammensetzung kein Konservierungsmittel enthält.

Die Verbindungen der Formel **1** sind aus der WO 02/32899 und aus der WO 91/04252 bekannt. Sie besitzen wertvolle pharmakologische Eigenschaften und können als hochwirksame Anticholinergika bei der Therapie von Atemwegserkrankungen, vorzugsweise bei der Therapie entzündlicher und/oder obstruktiver Atemwegserkrankungen, insbesondere bei der Therapie von Asthma oder COPD (chronic obstructive pulmonary disease = chronisch obstruktive Lungenerkrankung) einen therapeutischen Nutzen entfalten.

Es war Aufgabe der vorliegenden Erfindung, neue Verwendungen für die Verbindungen der Formel **1** bereitzustellen.

Überraschenderweise konnte gefunden werden, dass die Verbindungen der Formel **1** 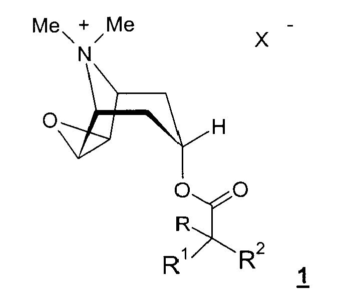 worin
- X ⁻: ein einfach negativ geladenes Anion,
- R: H, Methyl oder OH
- R¹ und R²: gleich oder verschieden, Phenyl oder Thienyl
bedeuten,
keimtötende Wirkung besitzen und insbesondere zur Abtötung von Mikroorganismen wie Bakterien und Pilzen verwendet werden können. Es konnte weiterhin gefunden werden, dass sich die Verbindungen der Formel **1** auch zur Herstellung eines Arzneimittels zur Behandlung von Atemwegsinfektionen eignen. Einen besonderen erfindungsgemäßen Vorteil stellt hierbei die Möglichkeit dar, mit nur einem einzigen Wirkstoff, nämlich einer Verbindung der Formel **1**, sowohl Asthma oder COPD als auch die häufig mit diesen Erkrankungen einhergehenden Atemwegsinfektionen behandeln zu können.

Die Applikation der Verbindungen nach Formel **1** erfolgt hierbei vorzugsweise auf inhalativem Wege. Hierbei können geeignete Inhalationspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten, aber auch Inhalationslösungen, insbesondere wässrige, ethanolische oder ethanolisch/wässrige Inhalationslösungen.

In US 2004/0209954 A1 wird beschrieben, dass bestimmte quaternäre Amine mit anticholinerger Wirkung wie Glycopyrrolat, Mepenzolat und Ipratropium eine antimikrobielle Aktivität besitzen, wobei jedoch keines der dort genannten Anticholinergika eine Scopin-Struktur besitzt. Die Scopin-Derivate der Formel **1**, die von den Anticholinergika Glycopyrrolat, Mepenzolat und Ipratropium strukturell weit entfernt sind, werden in US 2004/0209954 A1 jedoch nicht erwähnt.

Im Rahmen der vorliegenden Erfindung gelangen bevorzugt jene Verbindungen der Formel **1** zur Anwendung, in denen das Anion X ⁻ ausgewählt ist aus der Gruppe bestehend aus Chlorid, Bromid, lodid, Sulfat, Phosphat, Methansulfonat, Nitrat, Maleat, Acetat, Citrat, Fumarat, Tartrat, Oxalat, Succinat, Benzoat und p-Toluolsulfonat.

Bevorzugt gelangen die Salze der Formel **1** zur Anwendung, worin X ⁻ ein Anion ausgewählt aus der Gruppe Chlorid, Bromid, 4-Toluolsulfonat und Methansulfonat bedeutet.

Erfindungsgemäß besonders bevorzugt im Rahmen der vorliegenden Erfindung sind diejenigen Formulierungen, die die Verbindung der Formel **1**, in der X ⁻ für Bromid steht, enthalten.

Bezugnahmen auf die Verbindung der Formel **1** schließen im Rahmen der vorliegenden Erfindung stets alle möglichen amorphen und kristallinen Modifikationen dieser Verbindung mit ein. Bezugnahmen auf die Verbindung der Formel **1** schließen im Rahmen der vorliegenden Erfindung ferner alle möglichen Solvate und Hydrate, die von dieser Verbindung gebildet werden können, mit ein.

Besonders bevorzugt ist die Verwendung einer Verbindung nach Formel **1**,wobei R¹ und R² beide Phenyl, R Methyl und X⁻ Bromid bedeuten (2,2-Diphenylpropionsäurescopinester-Methobromid), zur Abtötung von Mikroorganismen sowie auch zur Herstellung eines Arzneimittels zur Behandlung von Atemwegsinfektionen. Diese Verbindung nach Formel **1**, wobei R¹ und R² beide Phenyl, R Methyl und X⁻ Bromid bedeuten, wird zur Abtötung von Mikroorganismen sowie auch zur Herstellung eines Arzneimittels zur Behandlung von Atemwegsinfektionen besonders bevorzugt in wässriger Lösung zur Inhalation und in der Konzentration von 0,1 bis 2,5 Gewichtsprozent, insbesondere von 0,2 bis 1,5 Gewichtsprozent eingesetzt.

Besonders bevorzugt ist weiterhin die Verwendung einer Verbindung nach Formel **1**, wobei R¹ und R² beide 2-Thienyl, R OH und X⁻ Bromid bedeutet (Tiotropiumbromid), zur Abtötung von Mikroorganismen sowie auch zur Herstellung eines Arzneimittels zur Behandlung von Atemwegsinfektionen. Diese Verbindung nach Formel **1**, wobei R¹ und R² beide 2-Thienyl, R Hydroxyl und X⁻ Bromid bedeuten, wird zur Abtötung von Mikroorganismen sowie auch zur Herstellung eines Arzneimittels zur Behandlung von Atemwegsinfektionen besonders bevorzugt in wässriger Lösung zur Inhalation und in der Konzentration von 0,005 bis 2,5 Gewichtsprozent, insbesondere von 0,01 bis 0,5 Gewichtsprozent eingesetzt.

Vorzugsweise werden die Verbindungen nach Formel **1** zur Abtötung von Mikroorganismen ausgewählt aus der Gruppe bestehend aus Pilzen und Bakterien eingesetzt. Die Verbindungen nach Formel **1** stärker bevorzugt zur Abtötung von Bakterien ausgewählt aus der Gruppe bestehend aus Escherichia coli (z.B. ATCC 8739), Pseudomonas aeruginosa (z.B. ATCC 9027), Staphylococcus aureus (z.B. ATCC 6538), Bacillus subtilis (z.B. ATCC 6633), Streptococcus viridans (z.B. M1040), Streptococcus pyogenes (z.B. ATCC12344 oder ATCC 19615), Neisseria spp., Streptococcus pneumoniae (z.B. ATCC 49 619), Corynebacterium spp., Pseudomonas spp. (z.B. Pseudomonas eeruginosa), Mycoplasma pneumoniae (z.B. ATCC 29342), Pseudomonas aeruginosa (z.B. ATCC 15442), Haemophilus influenzae (z.B. ATCC 51907 oder ATCC 33391), Chlamydophila pneumoniae (z.B. ATCC VR 1310), Moraxella catarrhalis (z.B. ATCC 25238 oder ATCC 8176), Enterobacteriaceae, Klebsiella pneumoniae (z.B. ATCC 10031 oder ATCC 13883) und Bacillus spp. eingesetzt oder zur Abtötung von Pilzen ausgewählt aus der Gruppe bestehend aus Candida albicans (z.B. ATCC 10231) und Aspergillus niger (z.B. ATCC 16404) eingesetzt. Besonders bevorzugt sind die Verbindungen nach Formel 1 zur Abtötung von Bakterien ausgewählt aus der Gruppe bestehend aus aus Escherichia coli (z.B. ATCC 8739), Staphylococcus aureus (z.B. ATCC 6538), Bacillus subtilis (z.B. ATCC 6633) und zur Abtötung von Pilzen ausgewählt aus der Gruppe bestehend aus Candida albicans (z.B. ATCC 10231) und Aspergillus niger (z.B. ATCC 16404) geeignet.

Eine weitere bevorzugte Ausführungsform der vorliegenden Erfindung betrifft die Verwendung der Verbindungen der Formel 1 zur Herstellung eines Arzneimittels zur Behandlung von Atemwegsinfektionen. Diese Atemwegsinfektionen werden vorzugsweise durch Bakterien ausgewählt aus der Gruppe bestehend aus Escherichia coli (z.B. ATCC 8739), Pseudomonas aeruginosa (z.B. ATCC 9027), Staphylococcus aureus (z.B. ATCC 6538), Bacillus subtilis (z.B. ATCC 6633), Streptococcus viridans (z.B. M1040), Streptococcus pyogenes (z.B. ATCC12344 oder ATCC 19615), Neisseria spp., Streptococcus pneumoniae (z.B. ATCC 49 619), Corynebacterium spp., Pseudomonas spp. (z.B. Pseudomonas eeruginosa), Mycoplasma pneumoniae (z.B. ATCC 29342), Pseudomonas aeruginosa (z.B. ATCC 15442), Haemophilus influenzae (z.B. ATCC 51907 oder ATCC 33391), Chlamydophila pneumoniae (z.B. ATCC VR 1310), Moraxella catarrhalis (z.B. ATCC 25238 oder ATCC 8176), Enterobacteriaceae, Klebsiella pneumoniae (z.B. ATCC 10031 oder ATCC 13883) und Bacillus spp, insbesondere aber durch Bakterien ausgewählt aus der Gruppe bestehend aus Escherichia coli (z.B. ATCC 8739), Pseudomonas aeruginosa (z.B. ATCC 9027), Staphylococcus aureus (z.B. ATCC 6538), Bacillus subtilis (z.B. ATCC 6633) verursacht. Diese Atemwegsinfektionen werden weiterhin vorzugsweise durch Pilze, insbesondere ausgewählt aus der Gruppe bestehend aus Candida albicans (z.B. ATCC 10231) und Aspergillus niger (z.B. ATCC 16404) verursacht. Besonders bevorzugt ist die Verwendung der Verbindungen der Formel **1** zur Herstellung eines Arzneimittels zur Behandlung von Atemwegsinfektionen, die durch die Pilze Aspergillus niger und Candida albicans oder durch ein Bakterium ausgewählt aus der Gruppe bestehend aus Pseudomonas aeroginosa oder Staphylococcus aureus, Bacillus subtilis und Escherichia coli hervorgerufen werden.

In einer weiteren bevorzugten Ausführungsform betrifft die Erfindung eine pharmazeutische Zusammensetzung enthaltend eine Verbindung der Formel **1** 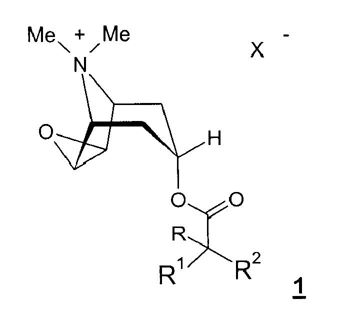 worin
- X ⁻: ein einfach negativ geladenes Anion,
- R: H, Methyl oder OH
- R¹ und R²: gleich oder verschieden, Phenyl oder Thienyl bedeuten,
gegebenenfalls in Form der einzelnen optischen Isomeren, Mischungen der einzelnen Enantiomeren oder Racemate, wobei die pharmazeutische Zusammensetzung kein zusätzliches Konservierungsmittel enthält.

Im Rahmen der vorliegenden Erfindung gelangen bevorzugt jene Verbindungen der Formel **1** zur Anwendung, in denen das Anion X ⁻ ausgewählt ist aus der Gruppe bestehend aus Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat.

Bevorzugt gelangen die Salze der Formel **1** zur Anwendung, worin X ⁻ ein Anion ausgewählt aus der Gruppe Chlorid, Bromid, 4-Toluolsulfonat und Methansulfonat bedeutet.

Erfindungsgemäß besonders bevorzugt im Rahmen der vorliegenden Erfindung sind diejenigen Formulierungen, die die Verbindung der Formel **1**, in der X ⁻ für Bromid steht, enthalten.

Die Applikation der Verbindungen nach Formel **1** erfolgt vorzugsweise auf inhalativem Wege. Hierbei können geeignete Inhalationspulver, die in geeignete Kapseln (Inhaletten) abgefüllt mittels entsprechender Pulverinhalatoren appliziert werden, zum Einsatz kommen. Alternativ dazu kann eine inhalative Anwendung auch durch Applikation geeigneter Inhalationsaerosole erfolgen. Hierzu zählen auch pulverförmige Inhalationsaerosole, die beispielsweise HFA134a, HFA227 oder deren Gemisch als Treibgas enthalten, und natürlich auch treibgashaltige oder treibgasfreie Inhalationslösungen der Verbindungen nach Formel **1** in einem Lösungsmittel ausgewählt aus der Gruppe bestehend aus Wasser, Ethanol und einem Wasser/Ethanol-Gemisch, wobei wässrige Inhalationslösungen bevorzugt und wässrige, treibgasfreie Inhalationslösungen besonders bevorzugt sind.

In einer besonders bevorzugten Ausführungsform betrifft die Erfindung eine pharmazeutische Zusammensetzung enthaltend eine Verbindung nach Formel **1**, worin R¹ und R² beide Phenyl, R Methyl und X⁻ Bromid bedeuten (2,2-Diphenylpropionsäurescopinester-Methobromid), gegebenenfalls in Form der einzelnen optischen Isomeren, Mischungen der einzelnen Enantiomeren oder Racemate, dadurch gekennzeichnet, dass die pharmazeutische Zusammensetzung kein Konservierungsmittel enthält. Diese pharmazeutische Zusammensetzung ist vorzugsweise eine wässrige, insbesondere eine treibgasfreie wässrige Aerosolformulierung enthaltend ein 2,2-Diphenylpropionsäurescopinester-Methobromid.

Hierbei ist das 2,2-Diphenylpropionsäurescopinester-Methobromid vorzugsweise in einer Konzentration von 0,1 bis 2,5 Gewichtsprozent, insbesondere in einer Konzentration von 0,2 bis 1,5 Gewichtsprozent in der wässrigen Aerosolformulierung anwesend. Der pH-Wert dieser bevorzugten Formulierung liegt erfindungsgemäß bevorzugt in einem Bereich von 2,5 und 6,5, vorzugsweise in einem Bereich von 3,0 bis 5,0, stärker bevorzugt im Bereich von 3,5 bis 4,5 , insbesondere im Bereich 3,6 bis 4,4.

In einer weiteren besonders bevorzugten Ausführungsform betrifft die Erfindung eine pharmazeutische Zusammensetzung enthaltend eine Verbindung nach Formel **1**, worin R¹ und R² 2-Thienyl, R Hydroxy und X⁻ Bromid bedeuten (Tiotropiumbromid), gegebenenfalls in Form der einzelnen optischen Isomeren, Mischungen der einzelnen Enantiomeren oder Racemate, dadurch gekennzeichnet, dass die pharmazeutische Zusammensetzung kein Konservierungsmittel enthält. Diese pharmazeutische Zusammensetzung ist vorzugsweise eine wässrige, insbesondere eine treibgasfreie Aerosolformulierung enthaltend Tiotropiumbromid.

Hierbei ist Tiotropiumbromid vorzugsweise in einer Konzentration von 0,005 bis 2,5 Gewichtsprozent, insbesondere in einer Konzentration von 0,01 bis 0,5 Gewichtsprozent in der wässrigen Aerosolformulierung anwesend. Der pH-Wert der erfindungsgemäßen Formulierung liegt zwischen 2,0 und 4,5, bevorzugt zwischen 2,5 und 3,5 und stärker bevorzugt zwischen 2,7 und 3,3 und besonders bevorzugt zwischen 2,7 und 3,2. Am stärksten bevorzugt sind pH-Werte mit einer oberen Grenze von 3,1.

Die bevorzugten wässrigen, treibgasfreien Aerosolformulierungen enthalten vorzugsweise zusätzlich die zum Einstellen des pH-Werts notwendige Säure(n).

Der pH-Wert wird durch Zugabe von pharmakologisch verträglichen Säuren eingestellt. Beispiele für diesbezüglich bevorzugte anorganische Säuren sind: Salzsäure, Bromwasserstoffsäure, Salpetersäure, Schwefelsäure und/oder Phosphorsäure. Beispiele für besonders geeignete organische Säuren sind: Ascorbinsäure, Zitronensäure, Äpfelsäure, Weinsäure, Maleinsäure, Bernsteinsäure, Fumarsäure, Essigsäure, Ameisensäure und/oder Propionsäure und andere. Bevorzugte anorganische Säuren sind Salzsäure, Schwefelsäure. Es können auch die Säuren verwendet werden, die bereits mit dem Wirkstoff ein Säureadditionssalz bilden. Unter den organischen Säuren sind Ascorbinsäure, Fumarsäure und Zitronensäure bevorzugt, wobei Zitronensäure am stärksten bevorzugt ist. Gegebenenfalls können auch Gemische der genannten Säuren eingesetzt werden, insbesondere in Fällen von Säuren, die neben ihren Säuerungseigenschaften auch andere Eigenschaften, z.B. als Geschmackstoffe oder Antioxidantien besitzen, wie beispielsweise Zitronensäure oder Ascorbinsäure. Als anorganische Säuren wird ausdrücklich Salzsäure genannt.

Gegebenenfalls können auch pharmakologisch verträgliche Basen zum genauen Austitrieren des pH-Wertes eingesetzt werden. Als Basen eignen sich beispielsweise Alkalihydroxide und Alkalicarbonate. Bevorzugtes Alkaliion ist Natrium. Werden solche Basen verwendet, ist darauf zu achten, daß auch die daraus resultierenden Salze, die dann in der fertigen Arzneimittelformulierung enthalten sind, mit der oben genannten Säure pharmakologisch akzeptabel ist.

Die bevorzugten wässrigen, treibgasfreien Aerosolformulierungen können außer dem Lösungsmittel Wasser und der Verbindung der Formel **1** zusätzlich einen Komplexbildner, insbesondere Natriumedetat, weitere Cosolventien sowie weitere Hilfs- und Zusatzstoffe wie z.B. Antioxidantien, oberflächenaktive Stoffe etc. mit Ausnahme von Konservierungsstoffen enthalten. In einer anderen Ausführungsform der wässrigen, treibgasfreien Aerosolformulierungen sind außer der Verbindung nach Formel **1** und dem Lösungsmittel Wasser keinerlei weitere Zusätze vorhanden.

Unter Komplexbildner werden im Rahmen der vorliegenden Erfindung bevorzugt Moleküle verstanden, die in der Lage sind Komplexbindungen einzugehen. Bevorzugt sollen durch diese Verbindungen Kationen, besonders bevorzugt metallische Kationen komplexiert werden.

Die erfindungsgemäßen Formulierungen enthalten als Komplexbildner bevorzugt Editinsäure (EDTA) oder bekannte Salze davon, z.B. Natrium-EDTA, bzw. Dinatrium-EDTA. Bevorzugt wird Natriumedetat und Dinatriumedetat, gegebenenfalls in Form ihrer Hydrate, besonders bevorzugt in Form ihrer Dihydrate eingesetzt.
Werden im Rahmen der erfindungsgemäßen Formulierungen Komplexbildner verwendet, so liegt deren Gehalt bevorzugt in einem Bereich von 5 bis 20 mg pro 100 ml, stärker bevorzugt in einem Bereich von 7 bis 15 mg pro 100 ml der erfindungsgemäßen Formulierung. Besonders bevorzugt enthalten die erfindungsgemäßen Formulierungen einen Komplexbildner, vorzugsweise Natriumedetat oder Dinatriumedetat bzw. eines ihrer Hydrate, in einer Menge von etwa 9 bis 12 mg pro 100 ml, insbesondere von etwa 10 mg pro 100 ml der erfindungsgemäßen Formulierung.

Analoges wie bereits für Natriumedetat und Dinatriumedetat ausgeführt, gilt auch für mögliche, mit EDTA oder seinen Salzen vergleichbare Zusatzstoffe, die komplexbildende Eigenschaften aufweisen und anstelle dessen verwendet werden können, wie beispielsweise Nitrilotriessigsäure und deren Salze.

Der erfindungsgemäßen Formulierung können weitere pharmakologisch verträgliche Hilfsstoffe zugesetzt werden. Unter Hilfs- und Zusatzstoffen wird in diesem Zusammenhang jeder pharmakologisch verträgliche und therapeutisch sinnvolle Stoff verstanden, der kein Wirkstoff ist, aber zusammen mit dem Wirkstoff in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. Stabilisatoren, Antioxidantien und/oder Konservierungsstoffe, die die Verwendungsdauer der fertigen Arzneimittelformulierung verlängern sowie Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid.

Zu den bevorzugten Hilfsstoffen zählen Antioxidantien, wie beispielsweise Ascorbinsäure, sofern nicht bereits für die Einstellung des pH-Werts verwendet, Vitamin A, Vitamin E, Tocopherole und ähnliche im menschlichen Organismus vorkommende Vitamine oder Provitamine.

Bevorzugte Co-Solventien sind solche, die Hydroxylgruppen oder andere polare Gruppen enthalten, beispielsweise Alkohole - insbesondere lsopropylalkohol, Glykole - insbesondere Propylenglykol, Polyethylenglykol, Polypropylenglykol, Glykolether, Glycerol, Polyoxyethylenalkohole und Polyoxyethylen-Fettsäureester, sofern sie nicht bereits das Lösungs- oder Suspensionsmittel sind.

Unter Hilfs- und Zusatzstoffen wird in diesem Zusammenhang jeder pharmakologisch verträgliche und therapeutisch sinnvolle Stoff verstanden, der einerseits kein Wirkstoff und andererseits kein Konservierungsmittel ist, aber zusammen mit dem Wirkstoff in dem pharmakologisch geeigneten Lösungsmittel formuliert werden kann, um die qualitativen Eigenschaften der Wirkstoffformulierung zu verbessern. Bevorzugt entfalten diese Stoffe keine oder im Kontext mit der angestrebten Therapie keine nennenswerte oder zumindest keine unerwünschte pharmakologische Wirkung. Zu den Hilfs- und Zusatzstoffen zählen z.B. oberflächenaktive Stoffe, wie z.B. Sojalecithin, Ölsäure, Sorbitanester, wie Sorbitantrioleat, Polyvinylpyrrolidon sonstige Stabilisatoren, Komplexbildner, Antioxidantien, Geschmackstoffe, Vitamine und/oder sonstige dem Stand der Technik bekannte Zusatzstoffe. Zu den Zusatzstoffen zählen auch pharmakologisch unbedenkliche Salze wie beispielsweise Natriumchlorid.

Die keimtötende Wirkung der Verbindungen nach Formel **1** kann durch Vergleich von Testlösungen ( = wäßrige Lösungen, die die Verbindung nach Formel **1** enthalten, mit Placebo-Lösungen ( = identische wäßrige Lösungen ohne die Verbindung nach Formel **1**) nach dem Testverfahren wie in European Pharmacopoeia 4 (2002), General Text 5.1.3, Efficiacy of Antimicrobial Preservation beschrieben nachgewiesen werden. Hierbei werden definierte Volumina der Testlösungen und der Placebo-Lösungen jeweils mit identischen Mengen einer Inoculationslösung eines bestimmen Mikroorganismus versetzt, homogenisiert und bei einer für das Wachstum des Mikroorganismus geeigneten Temperatur und Luftfeuchtigkeit inkubiert. Zu definierten Zeitpunkten (z.B. nach 1 Tag, 2 Tagen, 3 Tagen, 7 Tagen, 14 Tagen und 28 Tagen) wird dann die Anzahl der keimfähigen Organismen ("colony forming units") der inokulierten Formulierungen gemessen und jeweils für die Testlösung und für die Placebo-Lösung verglichen.

Zur Bestimmung der Anzahl der keimfähigen Organismen ("colony forming units") der inokulierten Formulierungen werden zu definierten Zeitpunkten Proben aus diesen Formulierungen entnommen und mit TSB-Medium (Medium A) enthaltend Inaktivator No. 5 (3% Tween 80, 0,3% Lecithin, 0,1% Histidin) verdünnt. Anschließend werden diese Verdünnungen einer Membranfiltration unterworfen. Zur Ermittlung der Anzahl der Bakterien oder der Pilze wurden die Membranfilter nacheinander auf TSA-Medium (Medium B) und auf Sabourraud Agar (Medium C) übertragen. Die TSA-Platten wurden für 3-5 Tage bei 30-35 °C und die Sabourraud-Agarplatten bei 20-25 °C jeweils für fünf Tage inkubiert. Anschließend werden die Kolonien auf beiden Platten ausgezählt.

## Patentansprüche

1. Verwendung einer Verbindung der Formel **1** 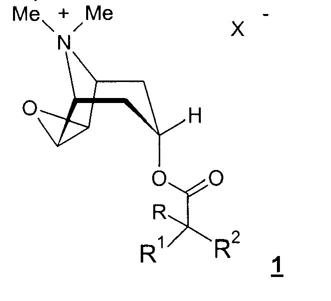 worin
X ⁻ ein einfach negativ geladenes Anion,
R H, Methyl oder OH
R¹ und R² gleich oder verschieden, Phenyl oder Thienyl
bedeuten,
gegebenenfalls in Form der einzelnen optischen Isomeren, Mischungen der einzelnen Enantiomeren oder Racemate, zur Herstellung eines Arzneimittels zur Abtötung von Mikroorganismen.

2. Verwendung einer Verbindung der Formel **1** 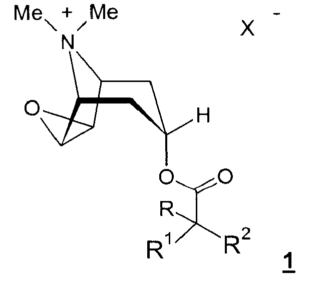 worin
X ⁻ ein einfach negativ geladenes Anion,
R H, Methyl oder OH
R¹ und R² gleich oder verschieden, Phenyl oder Thienyl
bedeuten,
gegebenenfalls in Form der einzelnen optischen Isomeren, Mischungen der einzelnen Enantiomeren oder Racemate, zur Herstellung eines Arzneimittels zur Behandlung von durch Mikroorganismen verursachten Atemwegsinfektionen.

3. Verwendung nach Anspruch 1 oder 2, **dadurch gekennzeichnet, dass** die Verbindung der Formel **1** inhalativ angewendet wird.

4. Verwendung nach Anspruch 1 bis 3, **dadurch gekennzeichnet, dass** in der Verbindung der Formel **1** R¹ und R² beide Phenyl, R Methyl und X ⁻ Bromid bedeutet.

5. Verwendung nach Anspruch 4, **dadurch gekennzeichnet, dass** die Verbindung nach Formel **1** in einer wässrigen Lösung in einer Konzentration von 0,1 bis 2,5 Gewichtsprozent anwesend ist.

6. Verwendung nach Anspruch 4, **dadurch gekennzeichnet, dass** die Verbindung nach Formel **1** in einer wässrigen Lösung in einer Konzentration von 0,2 bis 1,5 Gewichtsprozent anwesend ist.

7. Verwendung nach Anspruch 1 bis 3, **dadurch gekennzeichnet, dass** in der Verbindung der Formel **1** R¹ und R² 2-Thienyl und R OH bedeutet.

8. Verwendung nach Anspruch 7, **dadurch gekennzeichnet, dass** die Verbindung der Formel **1** in einer Konzentration von 0,005 bis 2,5 Gewichtsprozent anwesend ist.

9. Verwendung nach Anspruch 7, **dadurch gekennzeichnet, dass** die Verbindung der Formel **1** in einer Konzentration von 0,01 bis 0,5 Gewichtsprozent anwesend ist.

10. Verwendung nach Anspruch 1 bis 9, **dadurch gekennzeichnet, dass** die Mikroorganismen Pilze oder Bakterien sind.

11. Verwendung nach Anspruch 10, **dadurch gekennzeichnet, dass** die Mikroorganismen Bakterien sind und ausgewählt sind aus der Gruppe bestehend aus Escherichia coli, Pseudomonas aeruginosa, Staphylococcus aureus, Bacillus subtilis, Streptococcus viridans, Streptococcus pyogenes, Neisseria spp., Streptococcus pneumoniae, Corynebacterium spp., Pseudomonas spp., Mycoplasma pneumoniae, Pseudomonas aeruginosa, Haemophilus influenzae, Chlamydophila pneumoniae, Moraxella catarrhalis, Enterobacteriaceae, Klebsiella pneumoniae und Bacillus spp. eingesetzt.

12. Verwendung nach Anspruch 10, **dadurch gekennzeichnet, dass** die Mikroorganismen Pilze sind und ausgewählt sind aus der Gruppe bestehend aus Candida albicans und Aspergillus niger.

13. Pharmazeutische Zusammensetzung enthaltend eine Verbindung der Formel **1** 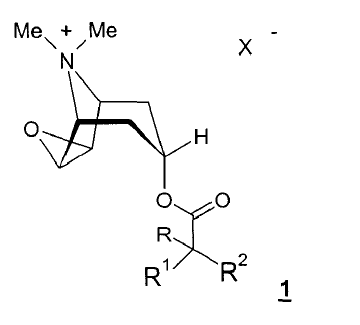 worin
X ⁻ ein einfach negativ geladenes Anion,
R H, Methyl oder OH
R¹ und R² gleich oder verschieden, Phenyl oder Thienyl
bedeuten,
gegebenenfalls in Form der einzelnen optischen Isomeren, Mischungen der einzelnen Enantiomeren oder Racemate, **dadurch gekennzeichnet, dass** die pharmazeutische Zusammensetzung kein Konservierungsmittel enthält.

14. Pharmazeutische Zusammensetzung nach Anspruch 13, **dadurch gekennzeichnet, dass** diese eine wässrige Aerosolformulierung ist.

15. Wässerige Aerosolformulierung zur Inhalation nach Anspruch 13 oder 14, **dadurch gekennzeichnet, dass** R¹ und R² beide Phenyl, R Methyl und X ⁻ Bromid bedeuten.

16. Wässerige Aerosolformulierung zur Inhalation nach Anspruch 15, **dadurch gekennzeichnet, dass** die Verbindung nach Formel **1** in einer Konzentration von 0,1 bis 2,5 Gewichtsprozent anwesend ist.

17. Wässerige Aerosolformulierung zur Inhalation nach Anspruch 15, **dadurch gekennzeichnet, dass** die Verbindung nach Formel 1 in einer Konzentration von 0,2 bis 1,5 Gewichtsprozent anwesend ist.

18. Wässerige Aerosolformulierung zur Inhalation nach Anspruch 13 oder 14, **dadurch gekennzeichnet, dass** R¹ und R² beide 2-Thienyl, R OH und X⁻ Bromid bedeuten.

19. Wässerige Aerosolformulierung zur Inhalation nach Anspruch 18, **dadurch gekennzeichnet, dass** die Verbindung nach Formel **1** in einer Konzentration von 0,005 bis 2,5Gewichtsprozent anwesend ist.

20. Wässerige Aerosolformulierung zur Inhalation nach Anspruch 18, **dadurch gekennzeichnet, dass** die Verbindung nach Formel **1** in einer Konzentration von 0,1 bis 0,5 Gewichtsprozent anwesend ist.
